# EUROPEAN PATENT SPECIFICATION

(11) **EP 3 720 516 B1**
(45) Date of publication and mention of the grant of the patent: **07.06.2023**
(21) Application number: 18829583.6
(22) Date of filing: 04.12.2018
(51) Int. Cl.: A61L 29/04, A61L 29/14

(54) **URINARY CATHETERS INCLUDING MECHANICAL PROPERTIES THAT ARE STABLE OVER A RANGE OF TEMPERATURES**
HARNKATHETER MIT ÜBER EINEN TEMPERATURBEREICH BESTÄNDIGEN MECHANISCHEN EIGENSCHAFTEN
CATHÉTERS URINAIRES COMPRENANT DES PROPRIÉTÉS MÉCANIQUES STABLES SUR UNE PLAGE DE TEMPÉRATURES

(30) Priority: 05.12.2017 US 201762594896 P
(43) Date of publication of application: 14.10.2020
(73) Proprietor: Hollister Incorporated, Libertyville, IL 60048 (US)
(72) Inventor: MONTES DE OCA BALDERAS, Horacio, Libertyville, IL 60048 (US); SELLERS, Brent, H., Libertyville, IL 60048 (US)
(74) Representative: FRKelly
(86) International application number: PCT/US2018/063857
(87) International publication number: WO 2019/113077

(56) References cited:
- EP-A1- 1 593 710
- EP-A2- 1 745 807

## Description

### CROSS REFERENCE TO RELATED APPLICATION

This application claims the benefit and priority of U.S. Patent Application Serial No. 62/594,896, filed December 5, 2017.

### TECHNICAL FIELD

The present disclosure generally relates to urinary catheters that have a catheter tube that maintains the mechanical properties/features over a range temperatures and materials for forming the same.

### BACKGROUND

Catheters are used to treat many different types of medical conditions and typically include an elongated catheter tube that is inserted into and through a passageway or lumen of the body. Catheters, and in particular intermittent catheters, are commonly used by those who suffer from various abnormalities of the urinary system, such as urinary incontinence. With the advent of intermittent catheters, individuals with urinary system abnormalities can self-catheterize (i.e., self-insert and self-remove intermittent catheters) several times a day.

Intermittent urinary catheters may be used in locations and climates that have significant temperature changes. For example, locations that have hot summers and cold winters. Also, catheter users may travel between hot and cold climates. Given the intimate nature and sensitivity of self-catheterization, some urinary catheter users may desire to have a consistent self-catheterization experience. One common issue that catheter users may face with self-catheterization is that in hotter temperatures or climates, the catheter tubes may become very soft which can change the user's experience and possibly hinder the user's ability to insert/advance the catheter. It is not uncommon for some catheter users to place catheters in a refrigerator prior to use to cool the catheter and increase the catheter's stiffness. In colder temperatures/climates the catheter tubes may become stiffer which can also change the user's experience and hinder the ability to insert/advance the catheter, especially in male users wherein the catheter must traverse the curved pathway of the urethra. It also is not uncommon for some catheter users to warm the catheter prior to use to increase the catheter's flexibility.

Therefore, there is a need for catheters that have catheter tubes in which the mechanical properties of the tube remain relatively stable over various temperatures and/or over a temperature range. EP1745807 discloses a medical device including a substrate made of a polymer blend comprising a polyolefin and a composition having molecules with active hydrogen(s), wherein a hydrophilic layer is adhered to the substrate by the covalent bonds to said active hydrogen(s) in the substrate.

### SUMMARY

The present invention relates to a urinary catheter, comprising: a catheter tube made from a polymeric material comprising a blend of (i) thermoplastic elastomer comprising poly(1-butene) and (ii) polyolefin; wherein the polymeric material has a tan δ peak at 0°C or less and a storage modulus greater than about 10 MPa in the temperature range of 0°C and 40°C measured at 1 Hz oscillation frequency.

### BRIEF DESCRIPTION OF THE FIGURES

Fig. 1 is a graph showing the change in storage modulus over temperature for materials of the Example.
Fig. 2 is a graph showing the tan δ over temperature for materials of the Example.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

The present application discloses urinary catheter tubes wherein the mechanical properties of the tubes, such as the viscoelastic properties, are relatively stable over a range of temperatures. The present application also discloses polymeric materials having such stable mechanical properties. The stiffness of the polymeric materials and the catheter tubes made therefrom may be relatively stable at various different temperatures or over specific ranges temperatures. That is, the catheter tubes and polymeric materials may have relatively small changes in stiffness and flexibility at different temperatures. This may be beneficial to intermittent catheter users that use catheters in both warm and cold climates. This is because, the stiffness/flexibility of the catheter tube remaining relatively the same regardless of the temperature in which it is used can aid in creating a consistent user experience. Depending on where a catheter user resides or where the user travels to, the user may encounter temperatures anywhere between 0°C and 40°C. The catheter tubes disclosed herein may or may not have a lubricious hydrophilic coating thereon any/or may be used with a gel-lubricant applied to the catheter prior to catheterization.

One feature of the mechanical stability of the material from which a catheter tube is made may be the difference in the change of the material's storage modulus over a temperature range. Another feature of the mechanical stability of the material from which the catheter tube is made may be the temperature of the tan delta (tan δ) peak of the material. Tan δ is defined as the ratio between the loss modulus and the storage modulus. The methods for measuring storage modulus and the temperature of the tan δ peak may, for example, employ a Dynamic Mechanical Analyzer. A TA Instruments DMA Q800, available from TA Instruments, Inc., of New Castle, Del.; equipped with a film clamp and Thermal Advantage software for data acquisition may be used for such data analysis. Many other types of DMA devices exist, and the use of dynamic mechanical analysis is well known to those skilled in the art of polymer and copolymer characterization. In one method of dynamic mechanical analysis the parameters may include oscillation frequency of 1 Hz, oscillation amplitude of 25.0, static force of 0.0100 N, forcetrack at 125.0% and minimum oscillation force at 0.02000 N. Furthermore, when measuring the mechanical properties of the materials that form a medical tube, such as a urinary catheter tube, the wall of the catheter tube may be cut into a rectangular sample having a length of 33 mm and a width of 6.5 mm. The mechanical properties may then be analyzed using the instruments described above. In one instance the rectangular sample of the material of the medical tube may have a thickness of 0.71 mm.

The urinary catheter includes a catheter tube made from a polymeric material having a tan δ peak at 0°C or less. In embodiments, the polymeric material may have a tan δ peak at -10°C or less, or have a tan δ peak between about -15°C and 0°C, or a tan δ peak of between about -15°C and -10°C, or a tan δ peak of between about -20°C and - 15°C.

In addition to the above-mentioned tan δ peaks, the polymeric material from which the catheter is made also has a storage modulus greater than about 10 MPa in the temperature range of 0°C and 40°C. In one embodiment, the polymeric material may have a storage modulus of greater than about 20 MPa in the temperature range of 0°C and 40°C or between about 20 MPa and about 50 MPa in the temperature range of 0°C and 40°C. In another embodiment, the polymeric material may have a storage modulus of greater than about 40 MPa in the temperature range of 0°C and 40°C or between about 40 MPa and about 200 MPa in the temperature range of 0°C and 40°C.

Another mechanical feature of the polymer material, which may be in addition to the above features, is that the increase of the storage modulus may be less than or equal to 500% when the material is cooled from 40°C to 0°C. In another embodiment, the storage modulus increases less than 300% when cooled from 40°C to 0°C. In another embodiment the storage modulus increases less than 200% when cooled from 40°C to 0°C. In yet another embodiment, the ratio of the storage modulus at 0°C to that of the storage modulus at 40°C is between about 5:1 and about 2:1. Percentage increase of the storage modulus shall be understood in this context as follows: storage modulus at low temperature (e.g. 0 °C) divided by storage modulus at high temperature (e.g. 40 °C) multiplied by 100.

The polymeric materials from which the urinary catheter tubes are made include a blend of (i) thermoplastic elastomer comprising poly(1-butene) and (ii) polyolefin; The blending may be by physical blending, compounding or any other suitable bending method. Such blends may then be extruded or molded by any suitable methods to form a catheter tube. The TPE is compounded with a polyolefin such as polypropylene or polyethylene. These blends in turn can also be compounded with solid fillers and/or liquid additives to impart other functionalities to the blend. When the polyolefin is polypropylene, it may be, for example, syndiotactic polypropylene or random copolymer polypropylene. In one embodiment, the blends may have a melting point of about 140°C.

The TPE may be a block copolymer. The block copolymer may be, for example, a styrenic block copolymer TPE. Such stryenic block copolymer TPEs may include styrenebutadiene-styrene (SBS) or styrene-ethylene-butylene-styrene (SEBS). Such SEBS and SBS TPE materials are commonly, but not exclusively, sold under the tradename Kraton^{®}. In one embodiment, the SEBS may have a styrene content of greater than 20 %, or between about 20 % and about 70 %, or between about 40 % and about 60 %. The TPE also may be a poly(1-butene) based plastomer, such as Purell KT MR 07, and/or copolymers thereof.

In one embodiment, the TPE and polyolefin blend may include TPE in the amount of between 5 wt% and 95 wt% of the polymeric material and the polyolefin may be between about 5 wt% and about 95 wt% of the polymeric material. In another embodiment, the TPE may be between about 40 wt% and about 70 wt% and the polyolefin may be between about 30 wt% and about 60 wt% or the TPE may be between about 45 wt% and about 65 wt% and the polyolefin may be between about 35 wt% and about 55 wt%. In yet another embodiment, the TPE may be about 50 wt% and the polyolefin may be about 50 wt%. For example, in one embodiment, the material of the catheter tube may be between about 40 wt% and about 60 wt% poly(1-butene) or copolymers thereof, and between about 40 wt% and about 60 wt% polypropylene.

### Example

The storage modulus and the tan δ the materials of various catheter tubes were measured using a TA Instruments Q-800 DMA and Thermal Advantage V7.5 Build 127 software. The parameters of the testing instrument were oscillation frequency of 1 Hz, oscillation amplitude of 25.0, static force of 0.0100 N, forcetrack at 125.0% and minimum oscillation force at 0.02000 N.

To measure the materials of the catheter tubes, a rectangular sample was cut from each of the tubes. The rectangular sample had the approximate dimensions of: length ~ 33 mm, width - 6.5 mm and thickness - 0.71 mm. The sample was then placed in a tension clamp and the measurements were taken.

Samples of materials were cut from five catheter tubes. Two of the catheter tubes (Samples 3 and 4) were made from materials in accordance with the present invention, while two catheter tubes were made from blends of thermoplastic elastomers (Samples 1 and 2, not according to the invention) and one catheter tube (Sample 5) was a commercially available catheter for comparative purposes.

**Table 1**

| Sample | Material of Catheter |
|---|---|
| 1 | Wittenburg PR12662 E Shore 80A : SEBS W (80A) |
| | Supplied by Wittenburg B.V. |
| 2 | Wittenburg PR12662 D2 Shore 90A : SEBS W (90A) |
| | Supplied by Wittenburg B.V. |
| 3 | 60 wt% Purell KT MR 07 / 40 wt% Purell RP270G : PB-1 (80A) |
| | Supplied by LyondellBasell |
| 4 | 45% Purell KT MR 07 / 55% Purell RP270G : PB-1 (90A) |
| | Supplied by LyondellBasell |
| 5 | Lofric^{®} Ch14 TPE catheter: Lofic^{®} |
| | Supplied by Wellspect/Dentsply IH AB |

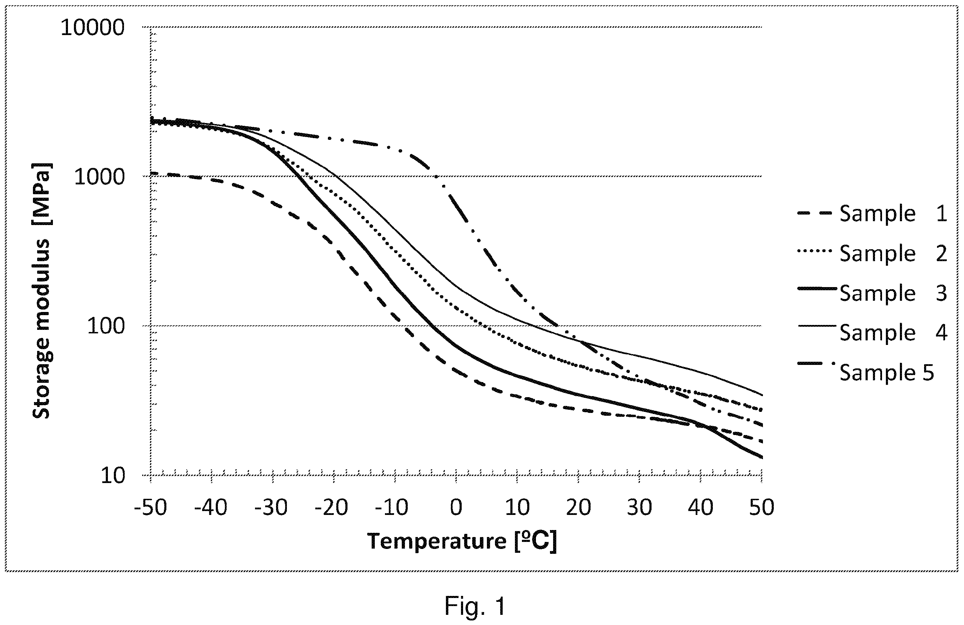

Table 2 below shows the storage modulus of each of the materials at 0°C and 40°C and the percent change of the storage modulus between these two temperatures. Fig. 1 includes a graph of the storage modulus over temperature.

**Table 2**

| | E' [Mpa] | E' [Mpa] | |
|---|---|---|---|
| | 0C | 40 C | % |
| Sample 1 | 50 | 21 | 138 |
| Sample 2 | 132 | 35 | 277 |
| Sample 3 | 73 | 21 | 248 |
| Sample 4 | 185 | 49 | 278 |
| Sample 5 | 636 | 30 | 2020 |

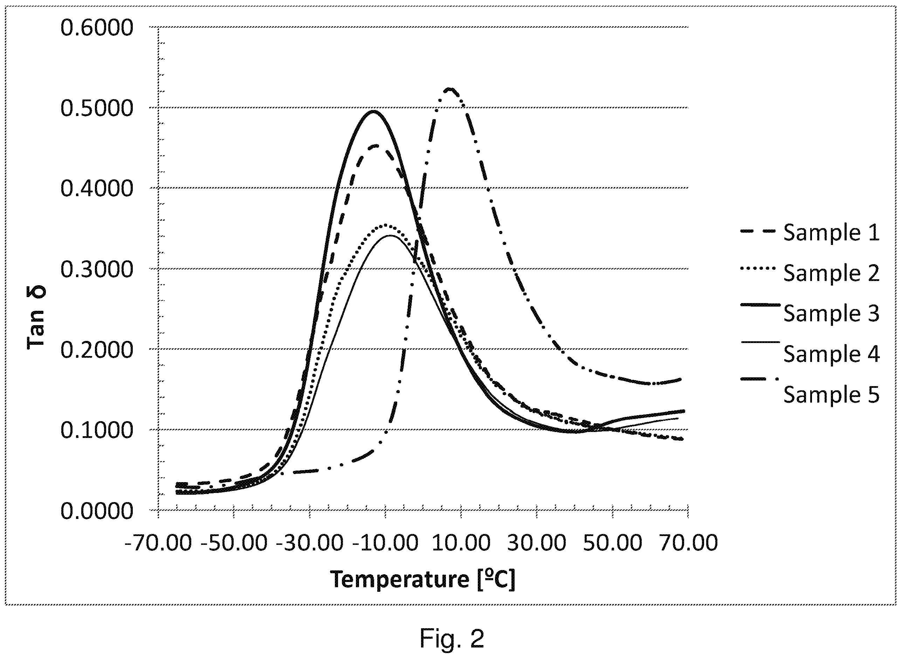

Fig. 2 includes a graph of the tan δ for each of the materials. As shown in the graph, each of Samples 1-4 has a tan δ peak at a temperature less than 0°C and Sample 5 has a tan δ peak at a temperature above 0°C.

## Claims

1. A urinary catheter, comprising:
a catheter tube made from a polymeric material comprising a blend of (i) thermoplastic elastomer comprising poly(1-butene) and (ii) polyolefin;
wherein the polymeric material has a tan δ peak at 0°C or less and a storage modulus greater than about 10 MPa in the temperature range of 0°C and 40°C measured at 1Hz oscillation frequency.

2. The urinary catheter of claim 1, wherein a ratio of the storage modulus at 0°C to the storage modulus at 40°C is between about 5 to 1 and about 2 to 1.

3. The urinary catheter of any one of claims 1 and 2, wherein the storage modulus increases less than 500% when the polymeric material of the catheter tube is cooled from 0°C to 40°C, and preferably less than 300%.

4. The urinary catheter of claim 1, wherein the poly(1-butene) is in a copolymer.

5. The urinary catheter of any one of claims 1-4, wherein the thermoplastic elastomer is between 5 wt% and 95 wt% of the polymeric material and the polyolefin is between about 5 wt% and about 95 wt% of the polymeric material.

6. The urinary catheter of any one of claims 1-4, wherein the thermoplastic elastomer is between about 40 wt% and about 70 wt% and the polyolefin is between about 30 wt% and about 60 wt%.

7. The urinary catheter of any one of claims 1-4, wherein the thermoplastic elastomer is between about 45 wt% and about 65 wt% and the polyolefin is between about 35 wt% and about 55 wt%

8. The urinary catheter of any one of claims 1-7, wherein the polyolefin comprises polypropylene.

9. The urinary catheter of claim 8, wherein the polypropylene comprises syndiotactic polypropylene and/or random copolymer polypropylene.

10. The urinary catheter of any one of claims 1-9, further including a lubricious hydrophilic coating disposed on an outer surface of the catheter tube.

11. The urinary catheter of any one of claims 1-10, wherein the melting temperature of the polymeric material is about 140°C.

## Patentansprüche

1. Harnkatheter, umfassend:
einen Katheterschlauch hergestellt aus einem Polymermaterial, umfassend eine Mischung aus (i) thermoplastischem Elastomer, das Poly(1-buten) umfasst, und (ii) Polyolefin;
wobei das Polymermaterial einen tan-5-Peak bei 0 °C oder weniger und einen Speichermodul größer als etwa 10 MPa in dem Temperaturbereich von 0 °C und 40 °C gemessen bei Oszillationsfrequenz von 1 Hz aufweist.

2. Harnkatheter nach Anspruch 1, wobei ein Verhältnis des Speichermoduls bei 0 °C zu dem Speichermodul bei 40 °C zwischen etwa 5 zu 1 und etwa 2 zu 1 beträgt.

3. Harnkatheter nach einem der Ansprüche 1 und 2, wobei der Speichermodul um weniger als 500 % zunimmt, wenn das Polymermaterial des Katheterschlauchs von 0 °C auf 40 °C gekühlt wird, und vorzugsweise um weniger als 300 %.

4. Harnkatheter nach Anspruch 1, wobei das Poly(1-buten) in einem Copolymer vorliegt.

5. Harnkatheter nach einem der Ansprüche 1 bis 4, wobei das thermoplastische Elastomer zwischen 5 Gew.-% und 95 Gew.-% des Polymermaterials beträgt und das Polyolefin zwischen etwa 5 Gew.-% und etwa 95 Gew.-% des Polymermaterials beträgt.

6. Harnkatheter nach einem der Ansprüche 1 bis 4, wobei das thermoplastische Elastomer zwischen etwa 40 Gew.-% und etwa 70 Gew.-% beträgt und das Polyolefin zwischen etwa 30 Gew.-% und etwa 60 Gew.-% beträgt.

7. Harnkatheter nach einem der Ansprüche 1 bis 4, wobei das thermoplastische Elastomer zwischen etwa 45 Gew.-% und etwa 65 Gew.-% beträgt und das Polyolefin zwischen etwa 35 Gew.-% und etwa 55 Gew.-% beträgt.

8. Harnkatheter nach einem der Ansprüche 1 bis 7, wobei das Polyolefin Polypropylen umfasst.

9. Harnkatheter nach Anspruch 8, wobei das Polypropylen syndiotaktisches Polypropylen und/oder Random-Copolymer-Polypropylen umfasst.

10. Harnkatheter nach einem der Ansprüche 1 bis 9, ferner beinhaltend eine gleitfähige hydrophile Beschichtung, die auf einer Außenfläche des Katheterschlauchs angeordnet ist.

11. Harnkatheter nach einem der Ansprüche 1 bis 10, wobei die Schmelztemperatur des Polymermaterials etwa 140 °C beträgt.

## Revendications

1. Cathéter urinaire, comprenant :
un tube de cathéter fabriqué à partir d'un matériau polymère comprenant un mélange de (i) élastomère thermoplastique comprenant du poly(1-butène) et (ii) polyoléfine ;
dans lequel le matériau polymère a un pic tan δ à 0°C ou moins et un module de stockage supérieur à environ 10 MPa dans la plage de températures de 0°C à 40°C mesuré à une fréquence d'oscillation de 1 Hz.

2. Cathéter urinaire selon la revendication 1, dans lequel un rapport entre le module de stockage à 0°C et le module de stockage à 40°C est compris entre environ 5 pour 1 et environ 2 pour 1.

3. Cathéter urinaire selon l'une quelconque des revendications 1 et 2, dans lequel le module de stockage augmente de moins de 500 % lorsque le matériau polymère du tube de cathéter est refroidi de 0°C à 40°C, et de préférence de moins de 300 %.

4. Cathéter urinaire selon la revendication 1, dans lequel le poly(1-butène) est dans un copolymère.

5. Cathéter urinaire selon l'une quelconque des revendications 1 à 4, dans lequel l'élastomère thermoplastique représente entre 5 % en poids et 95 % en poids du matériau polymère et la polyoléfine représente entre environ 5 % en poids et environ 95 % en poids du matériau polymère.

6. Cathéter urinaire selon l'une quelconque des revendications 1 à 4, dans lequel l'élastomère thermoplastique représente entre environ 40 % en poids et environ 70 % en poids et la polyoléfine représente entre environ 30 % en poids et environ 60 % en poids.

7. Cathéter urinaire selon l'une quelconque des revendications 1 à 4, dans lequel l'élastomère thermoplastique représente entre environ 45 % en poids et environ 65 % en poids et la polyoléfine représente entre environ 35 % en poids et environ 55 % en poids.

8. Cathéter urinaire selon l'une quelconque des revendications 1 à 7, dans lequel la polyoléfine comprend du polypropylène.

9. Cathéter urinaire selon la revendication 8, dans lequel le polypropylène comprend du polypropylène syndiotactique et/ou du polypropylène copolymère aléatoire.

10. Cathéter urinaire selon l'une quelconque des revendications 1 à 9, comportant en outre un revêtement hydrophile lubrifiant disposé sur une surface extérieure du tube de cathéter.

11. Cathéter urinaire selon l'une quelconque des revendications 1 à 10, dans lequel la température de fusion du matériau polymère est d'environ 140°C.
